# EUROPEAN PATENT SPECIFICATION

(11) **EP 3 854 327 B1**
(45) Date of publication and mention of the grant of the patent: **10.01.2024**
(21) Application number: 18934183.7
(22) Date of filing: 21.09.2018
(51) Int. Cl.: A61B 17/34, A61M 25/06, A61M 29/00, A61M 25/00

(54) **DILATOR**
DILATATOR
DILATATEUR

(43) Date of publication of application: 28.07.2021
(73) Proprietor: ASAHI INTECC CO., LTD., Seto-shi, Aichi 489-0071 (JP)
(72) Inventor: FUSEYA, Yukihiro, Seto-shi, Aichi 489-0071 (JP); YAGI, Masaru, Seto-shi, Aichi 489-0071 (JP)
(74) Representative: Prinz & Partner mbB
(86) International application number: PCT/JP2018/035091
(87) International publication number: WO 2020/059121

(56) References cited:
- EP-A1- 0 530 970
- WO-A1-01/19444
- WO-A1-2018/180209
- JP-A- 2001 218 851
- JP-A- 2002 177 289
- JP-A- 2012 100 827
- JP-A- 2014 524 807
- US-A1- 2009 281 386
- US-A1- 2014 046 357
- US-A1- 2017 007 230

## Description

### TECHNICAL FIELD

The present invention relates to a dilator.

### BACKGROUND ART

Dilators are known for expanding a hole formed on the wall of a patient's digestive tract and the like for the purpose of treatment. The distal end of the dilator is inserted into the hole formed on the wall, and the hole is expanded by pushing a tapered portion into the hole. Such a dilator is, for example, disclosed in Patent Literature 1. Patent Literature 2 discloses a dilator having an outer threadform.

### CITATION LIST

### Patent Literature

Patent Literature 1: Japanese Unexamined Patent Application Publication No. 2008-11867
Patent Literature 2: US 2009/0281386 A1.

### SUMMARY OF INVENTION

### TECHNICAL PROBLEMS

In such dilators, a sufficient propulsive force cannot be achieved at the tapered portion where the pushing resistance with respect to a hole or a constricted part increases, and the dilator could not sufficiently expand the hole in some cases. Therefore, a configuration can be considered where a spirally-arranged protruding portion is formed by winding a coil around the outer periphery of the dilator, and the dilator is advanced by ensuring a propulsive force is obtained due to a screw effect caused by rotation.

However, when a coil is only wound around the outer periphery of the dilator, the protruding portion that extends in a spiral may become displaced in a lengthwise axis direction.

The present invention has an object of providing a dilator that can suppress displacement, in a lengthwise axis direction, of a protruding portion that extends in a spiral.

### SOLUTION TO PROBLEM

The invention is defined by claim 1. Further embodiments of the invention are defined by the dependent claims. Methods do not form part of the claimed invention.

In order to achieve the object, a dilator according to an embodiment of the present invention comprises: a hollow shaft having an outer diameter that increases from a distal end toward a proximal end; and
a protruding portion that is provided on an outer periphery of the hollow shaft, and extends along the outer periphery of the hollow shaft in a spiral along a lengthwise axis direction of the hollow shaft; wherein the protruding portion has gaps between adjacent parts of the protruding portions along the lengthwise axis direction, a covering layer is provided that covers at least an outer peripheral surface of the hollow shaft, the outer peripheral surface being located in the gaps, and a top portion of the protruding portion is exposed.

Furthermore, the protruding portion may be provided so as to make contact with the outer peripheral surface of the hollow shaft, and the covering layer may be located between adjacent parts of the protruding portion, and make contact with an outer peripheral surface of the protruding portion.

Moreover, the covering layer may cover the outer peripheral surface of the hollow shaft, and the protruding portion may be provided on the covering layer.

Furthermore, the hollow shaft may comprise a first coil, in which one or more wires are wound into a hollow shape, and the protruding portion may comprise a second coil, in which one or more wires are wound around the outer peripheral surface of the hollow shaft.

According to the present invention, a dilator can be provided that can suppress displacement, in a lengthwise axis direction, of a protruding portion that extends in a spiral.

### BRIEF DESCRIPTION OF DRAWINGS

FIG. 1 is an overall view of a dilator according to an embodiment of the present invention.
FIG. 2 is a cross-sectional view of the vicinity of a boundary between a tapered portion and a proximal end portion of the dilator.
FIG. 3 is a cross-sectional view of the vicinity of a boundary between a tapered portion and a proximal end portion of a dilator according to a modification.
FIG. 4 is an overall view of a dilator according to a modification.
FIG. 5 is a diagram of a distal end portion of a dilator according to a modification.
FIG. 6 is a diagram of a distal end portion of a dilator according to a modification.
FIG. 7 is a diagram of a distal end portion of a dilator according to a modification.

### EMBODIMENTS OF THE INVENTION

Hereinafter, embodiments of the present invention will be described with reference to the drawings. Note that the dimensions of the dilator shown in the drawings are dimensions shown for the purpose of facilitating an understanding of the implementation details, and do not correspond to the actual dimensions.

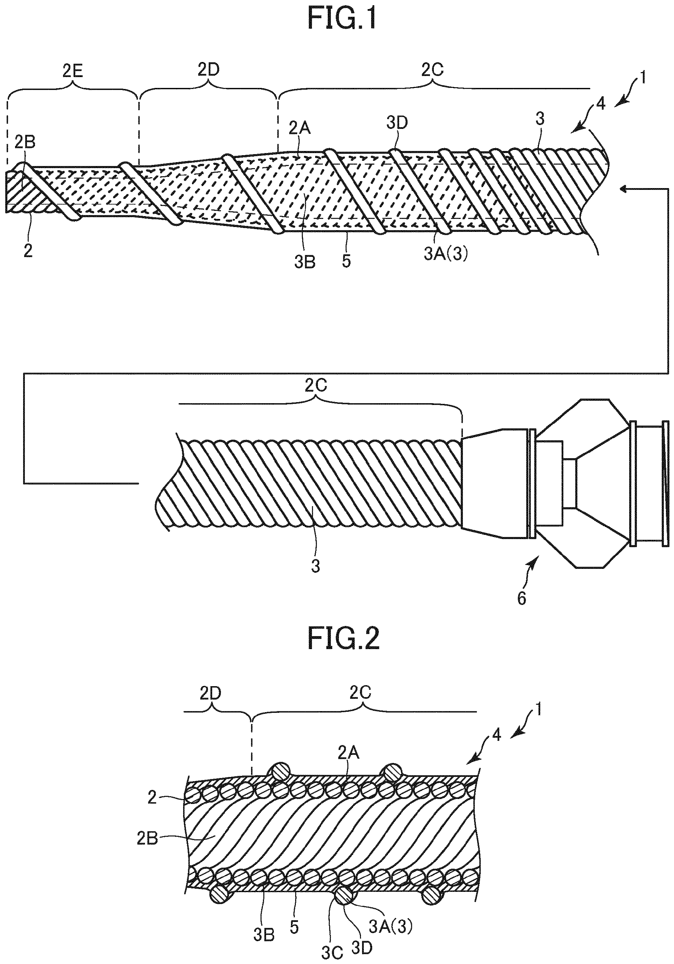

FIG. 1 is an overall view of a dilator 1 according to an embodiment of the present invention. FIG. 2 is a cross-sectional view of the vicinity of a boundary between a tapered portion 2D and a proximal end portion 2C of the dilator 1 according to the present embodiment.

Furthermore, in FIG. 1 and FIG. 2, the left side of the drawing is the distal end side (distal side) inserted into the body, and the right side is the proximal end side (hand side, proximal side) operated by an operator such as a physician.

In FIG. 1, the dilator 1 comprises: a multilayered body 4 configured by a first coil 2 formed by winding one or more metal wires into a hollow shape, and a second coil 3 formed by winding a single metal wire around an outer peripheral surface 2A of the first coil 2 in the opposite direction (Z-twisted) to the first coil 2 (S-twisted); a covering layer 5 that covers the outer peripheral surface 2A of the first coil 2; and a hollow connector 6 connected to the proximal end of the multilayered body 4. Note that the second coil 3 may be configured by a plurality of metal wires.

The wires constituting the first coil 2 and the second coil 3 are, for example, metal wires made of stainless steel or a superelastic alloy such as nickel-titanium, or are resin wires.

The first coil 2 is formed, for example, by winding ten metal wires made of stainless steel. The first coil 2 has a hollow shape, and is formed having a lumen 2B that passes through from the proximal end to the distal end. The first coil 2 includes a proximal end portion 2C, a tapered portion 2D, and a distal end portion 2E. The first coil 2 corresponds to a hollow shaft.

The proximal end portion 2C is located on the proximal end side of the dilator 1, and a connector 6 is connected to the proximal end thereof. Furthermore, the proximal end portion 2C has a substantially constant outer diameter from the proximal end to the distal end.

The tapered portion 2D is located on the distal end side of the proximal end portion 2C, extends from the distal end of the proximal end portion 2C toward the distal end side, and has an outer diameter that decreases toward the distal end side.

The distal end portion 2E is located on the distal end side of the tapered portion 2D, and extends from the distal end of the tapered portion 2D toward the distal end side. The distal end portion 2E has a substantially constant outer diameter from the proximal end to the distal end thereof. In this way, the outer diameter of the first coil 2, which is a hollow shaft, increases from the distal end toward the proximal end.

The second coil 3 is, for example, a single metal wire which is wound around the outer peripheral surface 2A of the first coil 2 in the opposite direction (Z-twisted) to the first coil 2 (S-twisted). Here, although the pitch of the metal wire is not particularly limited, the proximal end side represents a close winding in which a part of the metal wire are in contact with each other. The distal end side of the proximal end portion 2C, the tapered portion 2D, and the distal end portion 2E represent a sparse winding in which parts of the metal wire are separated from each other. As a result of the part of the second coil 3 that are wound with a gap (a sparsely wound part), a protruding portion 3A is formed that makes direct contact with the outer peripheral surface 2A of the first coil 2, and which extends in a spiral on the outer periphery of the first coil 2 along a lengthwise axis direction of the first coil 2. The protruding portion 3A has gaps 3B between adjacent parts of the protruding portion 3A (between adjacent parts of the metal wire) along the lengthwise axis direction of the first coil 2. The dilator 1 can also be advanced by a rotation operation the dilator 1 as a result of a screw action of the protruding portion 3A.

FIG. 2 is a cross-sectional view of the vicinity of a boundary between the tapered portion 2D and the proximal end portion 2C of the dilator 1.

The covering layer 5 is made of resin, and as shown in FIG. 2, covers the outer peripheral surface 2A of the first coil 2, which is located in the gaps 3B. That is to say, the covering layer 5 is located between adjacent parts of the protruding portion 3A, makes contact with an outer peripheral surface 3C of the protruding portion 3A, and covers the outer peripheral surface 3C of the protruding portion 3A. A top portion 3D of the protruding portion 3A is exposed to the outside from the covering layer 5. The top portion 3D is exposed to the outside from the covering layer 5, for example, by covering the entire periphery of the protruding portion 3A, including the top portion 3D, with resin, and then peeling off the resin near the top portion 3D. Examples of the resin constituting the covering layer 5 include biocompatible resin materials such as polyamide resins and fluororesins, and hydrophilic coating materials, and the thickness is, for example, 0.1 to 300 µm.

The length of the dilator in the present embodiment and the other embodiments described below is, for example, 2,000 mm, and preferably 1,600 mm to 2,500 mm; the length of the distal end portion 2E is, for example, 10 mm, and preferably 0 to 100 mm; and further, the length of the tapered portion 2D is, for example, 30 mm, and preferably 5 to 100 mm. The inner diameter at the distal end of the first coil 2 is, for example, 0.7 mm, and preferably 0.4 to 1.0 mm; and the inner diameter at the proximal end of the first coil 2 is, for example, 1.5 mm, and preferably 1.0 to 3.0 mm. The outer diameter at the distal end of the second coil 3 is, for example, 1.84 mm, and preferably 0.8 to 3.0 mm; and the outer diameter at the proximal end of the second coil 3 is, for example, 2.64 mm, and preferably 1.4 to 5.0 mm. Furthermore, the diameter of the metal wires of the first coil 2 is, for example, 0.21 mm, and preferably 0.1 to 0.5 mm; and the diameter of the metal wire of the second coil 3 is, for example, 0.36 mm, and preferably 0.1 to 0.5 mm.

The connector 6, which is a grip portion, is a portion that an operator uses to push the dilator into the body, or to perform a rotation operation. The distal end of the connector 6 is connected to the proximal end of the first coil 2 and the proximal end of the second coil 3. The connector 6 is made of resin, and has a hollow shape having a lumen which communicates with the lumen 2B of the first coil 2.

The dilator 1 of the present embodiment is provided with the covering layer 5, which covers the outer peripheral surface 2A of the first coil 2 located in the gaps 3B; therefore, it is possible to suppress displacement, in the lengthwise axis direction, of the protruding portion 3A that extends in a spiral. Furthermore, because the covering layer 5 is located between adjacent parts of the protruding portion 3A, and makes contact with the outer peripheral surface 3C of the protruding portion 3A, it is possible to suppress displacement of the protruding portion 3A in the lengthwise axis direction even further. The covering layer 5 enables the sliding properties of the dilator 1 to be improved, and the first coil 2 is capable of preventing pinching of the living tissue. Because the top portion 3D of the protruding portion 3A is exposed, compared to a case where the top portion 3D is covered by the covering layer 5, it is possible to improve the resistance to the abrasion that occurs with respect to the living tissue and the like at the time of rotation of the dilator 1.

Next, an example of the dilator when in use will be described. Methods are not claimed.

First, a target object is punctured with an introduction needle to form a hole. Then, after inserting a guide wire into a lumen of the introduction needle, the introduction needle is removed.

Next, the proximal end of the guide wire is inserted into the lumen of the dilator, and the dilator is inserted. Then, the dilator is pushed forward while rotating the shaft to expand the hole of the punctured portion. At this time, the tapered portion advances due to a screw action or the like of the spirally-arranged protruding portion due to the rotation operation of the shaft, and the hole can be smoothly expanded by the tapered portion.

Although embodiments of the present invention have been described above, the present invention is not limited to these embodiments, and various modifications can be made.

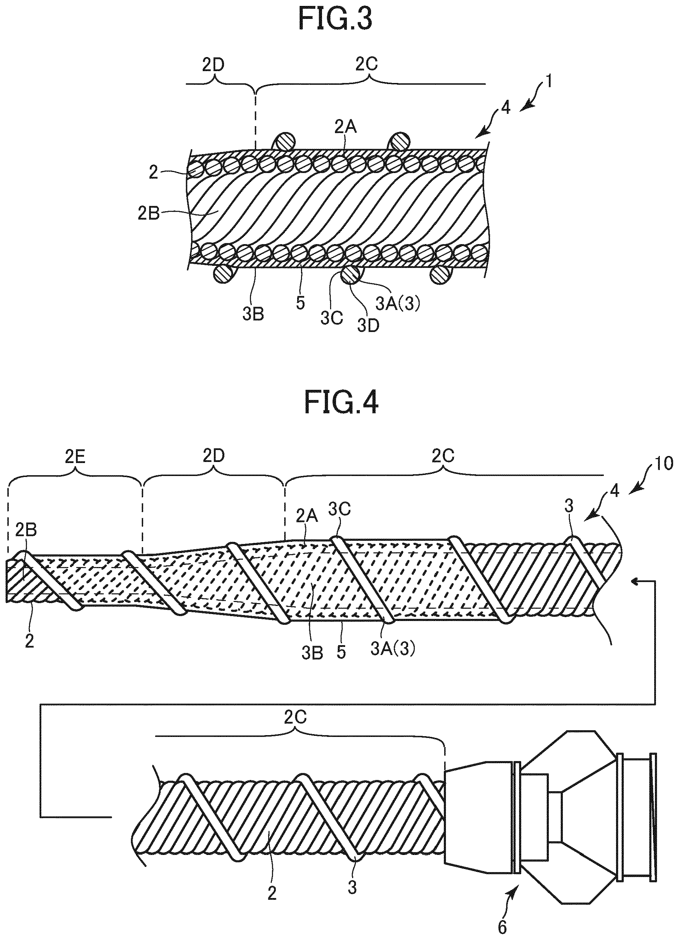

For example, as shown in FIG. 3, the covering layer 5 may cover the entire periphery of the outer peripheral surface 2A of the first coil 2, and the protruding portion 3A (second coil 3) may be provided on the covering layer 5. Specifically, the protruding portion 3A (second coil 3) is wound around the covering layer 5 while pressing the covering layer 5 toward the inner radial direction of the first coil 2 with the protruding portion 3A (second coil 3). As a result, a concave portion is formed on the covering layer 5 along the protruding portion 3A (second coil 3). Further, the structure becomes one in which the protruding portion 3A (second coil 3) is fitted into the concave portion. According to this configuration, because the covering layer 5 is located between adjacent parts of the protruding portion 3A, and makes contact with the outer peripheral surface 3C of the protruding portion 3A, it is possible to suppress displacement of the protruding portion 3A in the lengthwise axis direction, and to prevent pinching of the living tissue.

Furthermore, as shown in FIG. 4, the dilator 1 of the embodiment may be a dilator 10 in which the second coil 3 has gaps between adjacent parts along the axial direction of the first coil 3 up to the proximal end thereof.

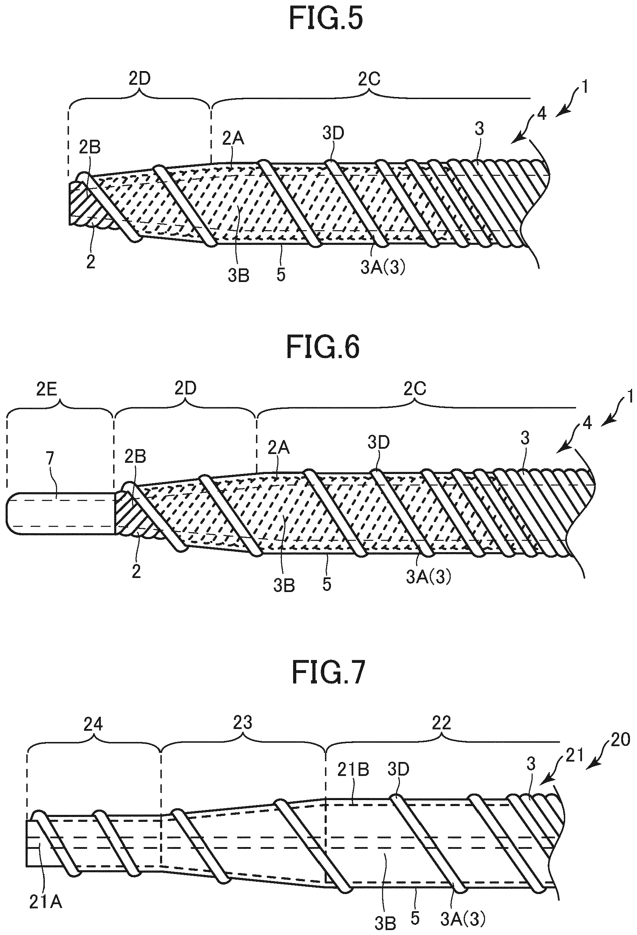

Moreover, as shown in FIG. 5, the first coil 2, which is a hollow shaft, does not have to have a distal end portion 2E, or as shown in FIG. 6, may have an approximately cylindrical and hollow leading-edge portion 7, which is formed by pouring a brazing material (a silver-tin brazing material, a gold-tin brazing material, or the like) into the distal end portion 2E of the first coil 2. In addition, a distal tip having the same shape as the leading-edge portion 7 may be provided on the distal end side of the tapered portion 2D instead of the distal end portion 2E (leading-edge portion 7). Also, the resin of the resin layer 5 may be provided in excess on the distal end portion 2E or on the distal end side of the tapered portion 2D, and a distal tip made of resin may be formed on the distal end side by the resin that has been provided in excess. Furthermore, a tip may be formed on the distal end portion 2E or on the distal end side of the tapered portion 2D using the resin of the resin layer 5 and a meltable resin material.

In addition, as shown in FIG. 7, a dilator 20 is also possible in which the first coil 2 is constituted by a hollow shaft 21 formed by casting or the like. The hollow shaft 21 has a hollow shape, and is formed having a lumen 21A that passes through from the proximal end to the distal end. Furthermore, the hollow shaft 21 includes a proximal end portion 22, a tapered portion 23, and a distal end portion 24, and the outer diameter increases from the distal end toward the proximal end. The material forming the hollow shaft 21 is not particularly limited as long as it ensures the softness of the tapered portion 23 and the distal end portion 24, and is biocompatible, and examples include stainless steel, superelastic alloy materials such as nickel-titanium alloy, and synthetic resins such as polyvinyl chloride resins, urethane resins, polyolefin resins, polyamide resins, and fluorine resins.

Further, the second coil 3 is wound around the outer peripheral surface 21B of the hollow shaft 21 in the same manner as in the embodiment above. That is to say, the second coil 3 is provided making direct contact with the outer peripheral surface 21B of the hollow shaft 21. The covering layer 5 covers the outer peripheral surface 21B of the hollow shaft 21, which is located in the gaps 3B of adjacent parts of the protruding portion 3A. That is to say, the covering layer 5 is located between adjacent parts of the protruding portion 3A, makes contact with the outer peripheral surface 3C of the protruding portion 3A, and covers the outer peripheral surface 3C of the protruding portion 3A. The top portion 3D of the protruding portion 3A is exposed to the outside from the covering layer 5.

The covering layer 5 may cover the entire periphery of the outer peripheral surface 21B of the hollow shaft 21, and the protruding portion 3A (second coil 3) may be provided on the covering layer 5. Specifically, the protruding portion 3A (second coil 3) is wound around the covering layer 5 while pressing the covering layer 5 toward the inner radial direction of the hollow shaft 21 with the protruding portion 3A (second coil 3). As a result, a concave portion is formed on the covering layer 5 along the protruding portion 3A (second coil 3). Further, the structure becomes one in which the protruding portion 3A (second coil 3) is fitted into the concave portion. According to this configuration, because the covering layer 5 is located between adjacent parts of the protruding portion 3A, and makes contact with the outer peripheral surface 3C of the protruding portion 3A, it is possible to suppress displacement of the protruding portion 3A in the lengthwise axis direction, and to prevent pinching of the living tissue.

The dilator 20 is also provided with the covering layer 5, which covers the outer peripheral surface 2A of the second coil 2 located in the gaps 3B; therefore, it is possible to suppress displacement, in the lengthwise axis direction, of the protruding portion 3A that extends in a spiral. Because the covering layer 5 is located between adjacent parts of the protruding portion 3A, and makes contact with the outer peripheral surface 3C of the protruding portion 3A, it is possible to suppress displacement of the protruding portion 3A in the lengthwise axis direction even further. Because the top portion 3D of the protruding portion 3A is exposed, compared to a case where the top portion 3D is covered by the covering layer 5, it is possible to improve the resistance to the abrasion that occurs with respect to the living tissue and the like at the time of rotation of the dilator 1.

Furthermore, the outer peripheral surface of the second coil 3, which is closely wound on the proximal end side of the first coil 2 or hollow shaft 21, may also be covered by a resin.

Moreover, in the embodiments above, although the first coil 2 was described as a hollow coil body formed from ten wires, the number of wires is not limited to ten, and may be one or more.

In the embodiment shown in FIG. 7, the surface of the hollow shaft 21 (including the portion between the shaft and the spirally-arranged protruding portion) may have various coatings. Examples of the coating include a protective film (a typical example being a plating film) on the surface of the hollow shaft 21, and a base film for improving the adhesion between the hollow shaft 21 and the second coil 3.

In the embodiments shown in FIG. 1 to FIG. 7, the spirally-arranged protruding portion preferably does not constitute a blade. The dilators of the present embodiments expand a pre-formed hole in a target object (an example being the wall of a digestive tract such as a patient's stomach). Therefore, if the spirally-arranged protruding portion constitutes a blade, the living tissue on the inner surface of the hole becomes damaged.

Therefore, the cross-sectional shape of the spirally-arranged protruding portion (for example, the shape of the cross-section taken orthogonally to the spiral direction of the spirally-arranged protruding portion 3A shown in FIG. 2) preferably does not have a corner portion having an acute angle on the radially outer end portion of the shaft. That is to say, the end portion preferably has a portion which is formed having, for example, a shape which contains a corner portion having an obtuse angle, or a curve (for example, a curve containing part of a circle or an ellipse).

### DESCRIPTION OF REFERENCE NUMERALS

1, 10, 20 Dilator
2 First coil
2A Outer peripheral surface
3 Second coil
3A Protruding portion
3B Gap
3D Top portion
5 Covering layer
21 Hollow shaft

## Claims

1. A dilator (10; 20) comprising:
a hollow shaft (21) having an outer diameter that increases from a distal end toward a proximal end; and
a protruding portion (3A) that is provided on an outer periphery of the hollow shaft (21), and extends along the outer periphery of the hollow shaft (21) in a spiral along a lengthwise axis direction of the hollow shaft (21);
the protruding portion (3A) has gaps (3B) between adjacent parts of the protruding portion (3A) along the lengthwise axis direction, **characterized in that**
a covering layer (5) is provided that covers at least an outer peripheral surface (2A) of the hollow shaft (21), said outer peripheral surface (2A) being located in the gaps (3B), and
a top portion(3D) of the protruding portion (3A) is exposed.

2. The dilator (10; 20) according to claim 1, wherein
the protruding portion (3A) is provided so as to make contact with the outer peripheral surface (2A) of the hollow shaft (21), and
the covering layer (5) is located between adjacent parts of the protruding portion (3A), and makes contact with an outer peripheral surface (2A) of the protruding portion (3A).

3. The dilator (10; 20) according to claim 1, wherein
the covering layer (5) covers the outer peripheral surface (2A) of the hollow shaft (21), and
the protruding portion (3A) is provided on the covering layer (5).

4. A dilator (10; 20) according to any one of claims 1 to 3, wherein
the hollow shaft (21) comprises a first coil (2), in which one or more wires are wound into a hollow shape (21), and
the protruding portion (3A) comprises a second coil (3), in which one or more wires are wound around the outer peripheral surface (2A) of the hollow shaft (21).

## Patentansprüche

1. Dilatator (10; 20) mit:
einem Hohlschaft (21) mit einem Außendurchmesser, der von einem distalen Ende zu einem proximalen Ende hin zunimmt, und
einem vorstehenden Teil (3A), der an einem Außenumfang des Hohlschafts (21) vorgesehen ist und entlang des Außenumfangs des Hohlschafts (21) in einer Spirale längs einer Längsachsenrichtung des Hohlschafts (21) verläuft,
wobei der vorstehende Teil (3A) Zwischenräume (3B) zwischen benachbarten Teilen des vorstehenden Teils (3A) längs der Längsachsenrichtung aufweist,
**dadurch gekennzeichnet, dass**
eine Deckschicht (5) vorgesehen ist, die zumindest eine Außenumfangsfläche (2A) des Hohlschafts (21) bedeckt, wobei die Außenumfangsfläche (2A) in den Zwischenräumen (3B) gelegen ist, und
ein oberer Teil (3D) des vorstehenden Teils (3A) freiliegt.

2. Dilatator (10; 20) nach Anspruch 1, bei dem
der vorstehende Teil (3A) so vorgesehen ist, dass er mit der Außenumfangsfläche (2A) des Hohlschafts (21) in Anlage gelangt, und
die Deckschicht (5) zwischen benachbarten Teilen des vorstehenden Teils (3A) liegt und mit einer Außenumfangsfläche (2A) des vorstehenden Teils (3A) in Anlage gelangt.

3. Dilatator (10; 20) nach Anspruch 1, bei dem
die Deckschicht (5) die Außenumfangsfläche (2A) des Hohlschafts (21) bedeckt und
der vorstehende Teil (3A) auf der Deckschicht (5) vorgesehen ist.

4. Dilatator (10; 20) nach einem der Ansprüche 1 bis 3, bei dem
der Hohlschaft (21) eine erste Spirale (2) umfasst, bei der ein oder mehrere Drähte zu einer Hohlform (21) gewickelt sind, und
der vorstehende Teil (3A) eine zweite Spirale (3) umfasst, bei der ein oder mehrere Drähte um die Außenumfangsfläche (2A) des Hohlschafts (21) gewickelt sind.

## Revendications

1. Dilatateur (10; 20), comprenant :
une tige creuse (21) présentant un diamètre extérieur qui augmente d'une extrémité distale à une extrémité proximale ; et
un tronçon en saillie (3A) qui est prévu sur une périphérie extérieure de la tige creuse (21) et qui s'étend en spirale le long de la périphérie extérieure de la tige creuse (21) selon un sens d'axe longitudinal de la tige creuse (21) ;
le tronçon en saillie (3A) présentant des fentes (3B) entre des parties adjacentes du tronçon en saillie (3A) selon le sens d'axe longitudinal,
**caractérisé en ce que**
il est prévu une couche de recouvrement (5) qui recouvre au moins une surface périphérique extérieure (2A) de la tige creuse (21), la surface périphérique extérieure (2A) étant agencée dans les fentes (3B), et
un tronçon supérieur (3D) du tronçon en saillie (3A) étant dégagé.

2. Dilatateur (10 ; 20) selon la revendication 1, dans lequel
le tronçon en saillie (3A) est prévu de manière à parvenir en contact avec la surface périphérique extérieure (2A) de la tige creuse (21), et
la couche de recouvrement (5) est agencée entre des parties adjacentes du tronçon en saillie (3A) et parvient en contact avec une surface périphérique extérieure (2A) du tronçon en saillie (3A).

3. Dilatateur (10 ; 20) selon la revendication 1, dans lequel
la couche de recouvrement (5) recouvre la surface périphérique extérieure (2A) de la tige creuse (21), et
le tronçon en saillie (3A) est prévu sur la couche de recouvrement (5).

4. Dilatateur (10 ; 20) selon l'une des revendications 1 à 3, dans lequel
la tige creuse (21) comprend un premier bobinage (2) dans lequel un ou plusieurs fils sont enroulés en une forme creuse (21), et
le tronçon en saillie (3A) comprend un deuxième bobinage (3) dans lequel un ou plusieurs fils sont enroulés autour de la surface périphérique extérieure (2A) de la tige creuse (21).
